# EUROPEAN PATENT SPECIFICATION

(11) **EP 1 774 009 B1**
(45) Date of publication and mention of the grant of the patent: **08.12.2010**
(21) Application number: 05757030.1
(22) Date of filing: 09.06.2005
(51) Int. Cl.: C12P 7/02, C12P 7/22

(54) **PREPARATION OF OPTICALLY ACTIVE ALCOHOLS WITH WHOLE-CELL CATALYSTS**
HERSTELLUNG VON OPTISCH AKTIVEN ALKOHOLEN MIT GANZZELLENKATALYSATOREN
PREPARATION D'ALCOOLS OPTIQUEMENT ACTIFS AVEC DES CATALYSEURS DE CELLULES ENTIERES

(30) Priority: 14.06.2004 DE 102004028407
(43) Date of publication of application: 18.04.2007
(73) Proprietor: Evonik Degussa GmbH, 45128 Essen (DE)
(72) Inventor: GRÖGER, Harald, 63450 Hanau (DE); MAY, Oliver, 52076 Aachen (DE); ROLLMANN, Claudia, 63755 Alzenau (DE); CHAMOULEAU, Francoise, 16340 L'Isle d'Espagna (FR); OROLOGAS, Nicolas, 54623 Thessalonoki (GR); DRAUZ, Karlheinz, 63589 Freigericht (DE)
(86) International application number: PCT/EP2005/006215
(87) International publication number: WO 2005/121350

(56) References cited:
- WO-A-2004/022764
- MATSUYAMA ET AL: "Practical application of recombinant whole-cell biocatalysts for the manufacturing of pharmaceutical intermediates such as chiral alcohols" ORGANIC PROCESS RESEARCH & DEVELOPMENT, vol. 6, 2002, pages 558-561, XP002344756 cited in the application
- GRÖGER ET AL: "Preparative asymmetric reduction of ketones in a biphasic medium with an (S)-alcohol dehydrogenase under in situ-cofactor-recycling with a formate dehydrogenase" TETRAHEDRON, vol. 60, January 2004 (2004-01), pages 633-640, XP004482876
- GRÖGER ET AL: "Practical asymmetric enzymatic reduction through diuscovery of a dehydrogenase-compatible biphasic reaction media" ORGANIC LETTERS, vol. 5, 2003, pages 173-176, XP001154877
- ERNST ET AL: "Enantioselective reduction of carbonyl compounds by whole-cell biotransformation, combining a formate dehydrogenase and a (R)-specific alcohol dehydrogenase" APPLIED MICROBIOLOGY AND BIOTECHNOLOGY, vol. 66, November 2004 (2004-11), pages 629-634, XP002344978
- WECKBECKER ET AL: "Improved synthesis of chiral alcohols with Escherichia coli cells co-expressing pyridine nucleotide transhydrogenase, NADP(+)-dependent alcohol dehydrogenase and NAD(+)-dependent formate dehydrogenase" BIOTECHNOLOGY LETTERS, vol. 26, November 2004 (2004-11), pages 1739-1744, XP002344979
- AMIDJOJO, M.H.: "Reaktionstechnische Untersuchungen zur asymmetrischen Synthese von chiralen Alkoholen mit Lactobacillus kefir" December 2004 (2004-12), pages 1-2, XP002344757 Retrieved from the Internet: URL:http://tumb1.biblio.tu-muenchen.de/pub l/diss/mw/2004/amidjojo.html> [retrieved on 2005-09-13]
- GRÖGER ET AL: "Enantioselective reduction of ketones with "designer cells" at high substrate concentrations: Highly efficient access to functionalized optically active alcohols" ANGEWANDTE CHEMIE, INTERNATIONAL EDITION, vol. 45, 2006, pages 5677-5681,

## Description

The present invention relates to a process for the preparation of optically active alcohols, starting from ketones, in the presence of a whole-cell catalyst comprising an alcohol dehydrogenase and also an enzyme capable of cofactor regeneration, which process is distinguished in that it is carried out at high substrate concentrations of >500 mM (without the addition of a cofactor), whereby the enzyme capable of cofactor regeneration is glucose dehydrogenase or malate dehydrogenase.

The preparation of optically active alcohols is of interest, for example, for the pharmaceuticals industry and the foodstuffs industry. A preferred form of preparation is to obtain the optically active alcohols by reduction of ketones in the presence of alcohol dehydrogenases. This enzymatic reduction of ketones has already been described in detail in the literature. For example, reference may be made here to the overview articles by M.-R. Kula, U. Kragl, Dehydrogenases in the Synthesis of Chiral Compounds in Stereoselective Biocatalysis (ed.: R. N. Patel), Dekker, 2000, Chapter 28, p. 839-866 and J. D. Stewart, Dehydrogenases and Transaminases in Asymmetric Synthesis in Current Opinion in Chemical Biology 2001, 5, 120-129. Also known in this connection are methods of "regenerating" the cofactor consumed during the reaction. A particularly interesting method of regenerating the cofactors NAD⁺ or NADP⁺ consists in using a second dehydrogenase enzyme, in particular a formate dehydrogenase or a glucose dehydrogenase. The use of a whole-cell catalyst for the reduction has proved to be a preferred form of implementation because - in contrast to the use of isolated enzymes in purified form or in the form of their crude extract - there are no additional costs for cell opening and enzyme purification. Likewise preferred is the use of recombinant expression systems, because high rates of expression can be achieved therewith. In comparison with non-recombinant cells, for example baker's yeast, such recombinant whole-cell catalysts can correspondingly be used in smaller amounts. A further advantage, in addition to.higher reaction rates, is the avoidance of undesirable secondary reactions by further dehydrogenase enzymes contained in wild-type cells. The advantages of the whole-cell method using microorganisms which have been genetically modified by means of recombinant DNA technology are described in detail, *inter alia,* in M. Kataoka, K. Kita, M. Wada, Y. Yasohara, J. Hasegawa, S. Shimizu, Appl. Microbiol. Biotechnol. 2003, 62, 437-445. In particular, *E. coli* cells that express an alcohol dehydrogenase and a glucose dehydrogenase for cofactor regeneration have proved to be especially suitable.

Reductions with high, commercially attractive substrate concentrations have proved to be a particular challenge. Using a whole-cell catalyst in which an ADH and a glucose dehydrogenase were present it has been possible in a two-phase reaction system to produce optically active alcohols even with high substrate concentrations of >500 mM. This has been demonstrated in particular for the preparation of (S)-4-chloro-3-hydroxybutanoic acid ethyl ester. However, it was necessary to add a cofactor, in this case NADP⁺, for the preparation of these alcohols. As described, for example, in N. Kizaki, Y. Yasohara, J. Hasegawa, M. Wada, M. Kataoka, S. Shimizu, Appl. Microbiol. Biotechnol. 2001, 55, 590-595, the added amount of NADP⁺ was in the region of about 0.001 mol. equivalent, based on substrate used. Because of the high price of NADP⁺, the added cofactor makes a significant contribution to the overall cost of the process even with these relatively small amounts of cofactor. Accordingly, a process that dispenses with the "external addition" of cofactor would be advantageous. whole-cell transformations (conversion of a substrate by means of whole cells), based on the activity of an alcohol dehydrogenase and a formate dehydrogenase as cofactor-regenerating enzyme, without the addition of cofactor, have recently been described in A. Matsuyama, H. Yamamoto, Y. Kobayashi, Organic Process Research & Development 2002, 6, 558-561. However, the substrate concentrations used were below 250 mM (e.g. 196 mM and 217 mM), and long reaction times of from 17 to 48 hours were required. Corresponding biotransformations with substantially higher substrate concentrations and short reaction times (less than 10 hours) are not known.

The significance of the addition of cofactor for the whole-cell method is also described in N. Itoh, M. Matsuda, M. Mabuchi, T. Dairi, J. Wang, Eur. J. Biochem. 2002, 269, 2394-2402. It was found here that a satisfactory conversion was achieved with the addition of only 0.5 mM, whereas scarcely any product formation took place without the addition of NAD⁺. In this connection *Itoh et al*. found that "the endogenous NAD+/NADH in the E. coli cells was insufficient for a smooth reaction", accompanied by the necessity for the "eternal" addition of cofactor.

The object of the present invention was, therefore, to develop a rapid, simple, inexpensive and effective process for the preparation of optically active alcohols from ketones.

The object has been achieved according to the invention by a process for the preparation of optically active alcohols by reduction of ketones in the presence of a whole-cell catalyst comprising an alcohol dehydrogenase and also an enzyme capable of cofactor regeneration, **characterised in that** the enzyme capable of co-factor regeneration is glucose dehydrogenase or malate dehydrogenase and that the conversion of a substrate concentration of at least 500 mM per starting volume of aqueous solvent used is carried out without the addition of an "external" cofactor. According to the invention this is to be understood as meaning that at least 500 mM of the substrate are converted by means of the described process per starting volume of aqueous solvent (including buffer system) used. It can be left open whether the at least 500 mM of substrate are actually achieved as concentration in the reaction mixture, or whether a substrate concentration of at least 500 mM is converted in total, based on the starting volume of aqueous solvent. The process is furthermore particularly suitable for the reduction of ketones using substrate concentrations of >500 mM, preferably >1000 mM and very preferably >1500 mM of ketone.

However, very particular preference is given to the variant in which a substrate concentration of at least 500 mM of ketone is actually provided for the conversion. The concentrations referred to here relate to concentrations of the substrate (ketone), based on the starting volume of aqueous solvent, that are actually achieved in the batch, it being immaterial when this starting concentration is achieved in the course of the period of incubation of a whole-cell catalyst that is used. The ketone can be used in these concentrations in the form of a batch directly at the start of a whole-cell batch, or a whole-cell catalyst can first be employed to a particular optical density, before the ketone is added. Likewise, the ketone can first be used in lower concentrations and added in the course of the incubation period of the cell batch to concentrations as indicated. According to the invention, however, a concentration of substrate (ketone) of at least 500 mM is achieved in the cell batch at least once during the conversion of the substrate to the desired alcohol.

Using this process, high to very high conversions to the corresponding optically active alcohol of at least 80%, especially >90% and very preferably >95% are surprisingly achieved at high substrate concentrations of at least 500 mM of ketone, especially >750 mM and very preferably >1000 mM even without the addition of an "external" cofactor. This was not to be expected on the basis of the conversions known hitherto and the known problems of the diffusion of the cofactor as a result of the permeabilisation of the cell membrane under the reaction conditions. This is additionally especially surprising because, at the high substrate concentrations of at least 500 mM of hydrophobic ketone component and in view of the low cell concentrations of the biocatalyst of <75 g/l, preferably <50 g/l, permeabilisation of the cell membrane should occur to a particularly great extent, accompanied by a loss of intracellular cofactor by "washing out" of the cofactor into the reaction medium.

The addition of the ketone can be carried out in any desired manner. Preferably, the total amount of ketone is added at the beginning ("batch" method), or alternatively it is added in metered amounts. It is also possible to employ continuous addition ("continuous feed-in process").

According to the invention, the process described here for the preparation of optically active alcohols is used. The conversion of ketones to optically active alcohols with the aid of alcohol dehydrogenases is known in principle to the person skilled in the art (see the literature references mentioned above). In order to obtain optically active alcohols it is particularly preferred to use ketones whose substituents are different from one another. The optically active alcohols which can be prepared from the corresponding ketones according to the invention can be subsumed under the following general formula 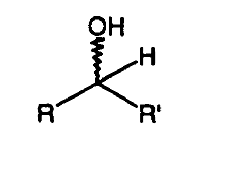 in which the R and R'-radicals are different from one another and are (C₁-C₈)-alkyl, (C₁-C₈)-alkoxy, (C₂-C₈)-alkoxyalkyl, wherein the aforementioned radicals may be mono- or poly-substituted by halogens and/or by radicals containing N, O, P, S, Si atoms, or are (C₃-C₈)-Cycloalkyl, which may be substituted by one or more halogens and/or radicals containing N, O, P, S, Si atoms and/or may contain N, O, P, S atoms in the ring or are HO-(C₁-C₈)-alkyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl-(C₆-C₁₈)-aryl, (C₁-C₈)-alkyl-(C₃-C₁₈)-heteroaryl, (C₁-C₈)-alkyl-(C₃-C₈)-cycloalkyl, (C₃-C₈)-cycloalkyl-(C₁-C₈)-alkyl, wherein (C₁-C₈) alkyl radical and (C₃-C₈)-cycloalkyl radical have the meaning indicated above.

The concentration of biocatalyst is not more than 75 g/l, in a preferred embodiment up to 50 g/l, preferably up to 25 g/l and particularly preferably up to 15 g/l, g being based on g of bio wet mass (BWM). The biocatalyst is to be understood as being especially a whole-cell catalyst.

In a preferred embodiment, the conversion of the ketone to the desired optically active alcohol is carried out without the addition of an organic solvent. This is intended to mean that no organic solvent is added to the batch containing the biocatalyst.

It is additionally preferred to carry out the conversion in a cell suspension of the suitable whole-cell catalyst, it being possible for the ketone used likewise to be present in the form of a suspension in the cell suspension or in the form of an emulsion or solution in the cell suspension.

For the present invention, one of the genes is a gene for an alcohol dehydrogenase. The person skilled in the art is likewise free to chose the genes that code for such an alcohol dehydrogenase. Examples of alcohol dehydrogenases that have proved to be preferable are alcohol dehydrogenases from a Lactobacillus strain, especially from *Lactobacillus kefir* and *Lactobacillus brevis,* or alcohol dehydrogenases from a *Rhodococcus* strain, especially from *Rhodococcus erythropolis* and *Rhodococcus ruber,* or alcohol dehydrogenases from an *Arthrobacter* strain, especially from *Arthrobacter paraffineus.*

A further gene that is used for the present invention is a gene that codes for a glucose dehydrogenases, preferably a glucose dehydrogenase from *Bacillus, Thermoplasma* and *Pseudomonas* strains, or malate dehydrogenases ("malic enzyme"), preferably a malic enzyme from *Sulfolobus, Clostridium, Bacillus* and *Pseudomonas* strains as well as from *E. coli,* especially *E. coli* K12.

According to the present invention, a "whole-cell catalyst" is to be understood as being an intact cell in which at least one gene is expressed that is able to catalyse the conversion according to the invention of a substrate to a product. According to the invention, the intact cell is capable of expressing an alcohol dehydrogenase and a dehydrogenase capable of cofactor regeneration. The whole-cell catalyst is preferably a genetically modified microorganism adapted to the requirements of the desired conversion. Preference is given as particularly suitable whole-cell catalysts to the two whole-cell catalysts described in the experimental part.

For the whole-cell catalyst, containing an alcohol dehydrogenase and an enzyme capable of cofactor regeneration as defined above, all known cells are suitable. There may be mentioned as microorganisms in this connection organisms such as, for example, yeasts such as *Hansenula polymorpha, Pichia sp*., *Saccharomyces cerevisiae*, prokaryotes, such as *E. coli, Bacillus subtilis,* or eukaryotes, such as mammalian cells, insect cells or plant cells. The cloning methods are well known to the person skilled in the art (Sambrook, J.; Fritsch, E. F. and Maniatis, T. (1989), Molecular cloning: a laboratory manual, 2nd ed., Cold Spring Harbor Laboratory Press, New York). *E. coli* strains are preferably to be used for this purpose. Very particular preference is given to: E. coli XL1 Blue, NM 522, JM101, JM109, JM105, RR1, DH5α, TOP 10- , HB101, BL21 codon plus, BL21 (DE3) codon plus, BL21, BL21 (DE3), MM294. Plasmids with which the gene construct containing the nucleic acid according to the invention is preferably cloned into the host organism are likewise known to the person skilled in the art (see also PCT/EP03/07148; see below).
Suitable plasmids or vectors are in principle any forms available to the person skilled in the art for this purpose. Such plasmids and vectors can be found, for example, in Studier *et al*. (Studier, W. F.; Rosenberg A. H.; Dunn J. J.; Dubendroff J. W.; (1990), Use of the T7 RNA polymerase to direct expression of cloned genes, Methods Enzymol. 185, 61-89) or the brochures of Novagen, Promega, New England Biolabs, Clontech or Gibco BRL. Further preferred plasmids and vectors can be found in: Glover, D. M. (1985), DNA cloning: a practical approach, Vol. I-III, IRL Press Ltd., Oxford; Rodriguez, R.L. and Denhardt, D. T (eds) (1988), Vectors: a survey of molecular cloning vectors and their uses, 179-204, Butterworth, Stoneham; Goeddel, D. V. (1990), Systems for heterologous gene expression, Methods Enzymol. 185, 3-7; Sambrook, J.; Fritsch, E. F. and Maniatis, T. (1989), Molecular cloning: a laboratory manual, 2nd ed., Cold Spring Harbor Laboratory Press, New York.
Plasmids with which the gene constructs containing the nucleic acid sequences under consideration can very preferably be cloned into the host organism are or are based on: pUC18/19 (Roche Biochemicals), pKK-177-3H (Roche Biochemicals), pBTac2 (Roche Biochemicals), pKK223-3 (Amersham Pharmacia Biotech), pKK-233-3 (Stratagene) or pET (Novagen).

In a further embodiment of the process according to the invention, the whole-cell catalyst is preferably pretreated before use in such a manner that the permeability of the cell membrane to the substrates and products is increased compared with the intact system. Particular preference is given to a process in which the whole-cell catalyst is pretreated, for example, by freezing and/or treatment with toluene.

According to the invention, the process is carried out without the addition of an "external" cofactor. This means that it is not necessary to add additional cofactor to the whole-cell batch, because the cells themselves already contain and are able to use a cofactor suitable for the conversion reaction. Cofactors suitable for the conversion are to be understood as being those to which electrons can be transferred, such as, for example, the NAD(P)+ H⁺ and the FADH₂ system.

The process according to the invention can be carried out at any reaction temperatures suitable for the host organism used. A particularly suitable reaction temperature is considered to be a reaction temperature that is from 10 to 90°C, preferably from 15 to 50°C and particularly preferably from 20 to 35°C.

The person skilled in the art is also free to choose the pH value of the reaction, it being possible to carry out the reaction both at a fixed pH value and with variation of the pH value within a pH range. The pH value is chosen taking into account in particular the needs of the host organism that is used. Preferably, the reaction is carried out at a pH value from pH 5 to 9, preferably from pH 6 to 8 and particularly preferably from pH 6.5 to 7.5.

The conversion of the substrate used to the desired product is carried out in cell culture using a suitable whole-cell catalyst. A suitable nutrient medium is employed according to the host organism that is used. The media suitable for the host cells are generally known and commercially available. It is additionally possible to add to the cell cultures conventional additives such as, for example, antibiotics, growth-promoting agents such as, for example, serums (foetal calf serum, etc.) and similar known additives.

(C₁-C₈)-Alkyl radicals are to be regarded as being methyl, ethyl, n-propyl, isopropyl, n-butyl, isobutyl, sec-butyl, *tert*-butyl, pentyl, hexyl, heptyl or octyl, including all their bond isomers.
The radical (C₁-C₈)-alkoxy corresponds to the radical (C₁-C₈)-alkyl, with the proviso that it is bonded to the molecule via an oxygen atom.
(C₂-C₈)-Alkoxyalkyl means radicals in which the alkyl chain is interrupted by at least one oxygen function, wherein two oxygen atoms may not be bonded to one another. The number of carbon atoms indicates the total number of carbon atoms contained in the radical.

The radicals just described may be mono- or poly-substituted by halogens and/or by radicals containing N, O, P, S, Si atoms. These are especially alkyl radicals of the above-mentioned type, which contain one or more of these hetero atoms in their chain or which are bonded to the molecule via one of these hetero atoms.

(C₃-C₈)-Cycloalkyl is understood as being cyclopropyl, cyclobutyl, cyclopentyl, cyclohexyl or cycloheptyl radicals, etc.. These radicals may be substituted by one or more halogens and/or radicals containing N, O, P, S, Si atoms and/or may contain N, O, P, S atoms in the ring, such as, for example, 1-, 2-, 3-, 4-piperidyl, 1-, 2-, 3-pyrrolidinyl, 2-, 3-tetrahydrofuryl, 2-, 3-, 4-morpholinyl.

A (C₃-C₈)-cycloalkyl-(C₁-C₈)-alkyl radical denotes a cycloalkyl radical as described above which is bonded to the molecule via an alkyl radical as indicated above.

Within the scope of the invention, (C₁-C₈)-acyloxy means an alkyl radical as defined above which has not more than 8 carbon atoms and is bonded to the molecule via a COO function.

Within the scope of the invention, (C₁-C₈)-acyl means an alkyl radical as defined above which has not more than 8 carbon atoms and is bonded to the molecule via a CO function.

A (C₆-C₁₈)-aryl radical is understood as being an aromatic radical having from 6 to 18 carbon atoms. Such radicals include in particular compounds such as phenyl, naphthyl, anthryl, phenanthryl, biphenyl radicals or systems of the above-described type fused to the molecule in question, such as, for example, indenyl systems, which may optionally be substituted by (C₁-C₈)-alkyl, (C₁-C₈)-alkoxy, NR¹R², (C₁-C₈)-acyl, (C₁-C₈)-acyloxy.

A (C₇-C₁₉)-aralkyl radical is a (C₆-C₁₈)-aryl radical bonded to the molecule via a (C₁-C₈)-alkyl radical.

Within the scope of the invention, a (C₃-C₁₈)-heteroaryl radical denotes a five-, six- or seven-membered aromatic ring system of from 3 to 18 carbon atoms which contains hetero atoms such as, for example, nitrogen, oxygen or sulfur in the ring. Such heteroaromatic compounds are regarded as being in particular radicals such as 1-, 2-, 3-furyl, 1-, 2-, 3-pyrrolyl, 1-, 2-, 3-thienyl, 2-, 3-, 4-pyridyl, 2-, 3-, 4-, 5-, 6-, 7-indolyl, 3-, 4-, 5-pyrazolyl, 2-, 4-, 5-imidazolyl, acridinyl, quinolinyl, phenanthridinyl, 2-, 4-, 5-, 6-pyrimidinyl.

A (C₄-C₁₉)-heteroaralkyl is understood as being a heteroaromatic system corresponding to the (C₇-C₁₉)-aralkyl radical.

Suitable halogens (Hal) are fluorine, chlorine, bromine and iodine.

The term aqueous solvent is understood as meaning water or a solvent mixture consisting mainly of water with watersoluble organic solvents such as, for example, alcohols, especially methanol or ethanol, or ethers, such as THF or dioxane.

### Figures:

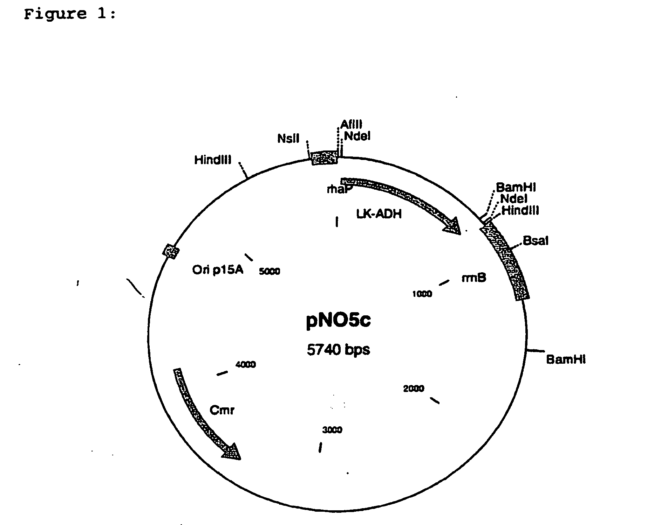

Figure 1 shows the plasmid map of plasmid pNO5c

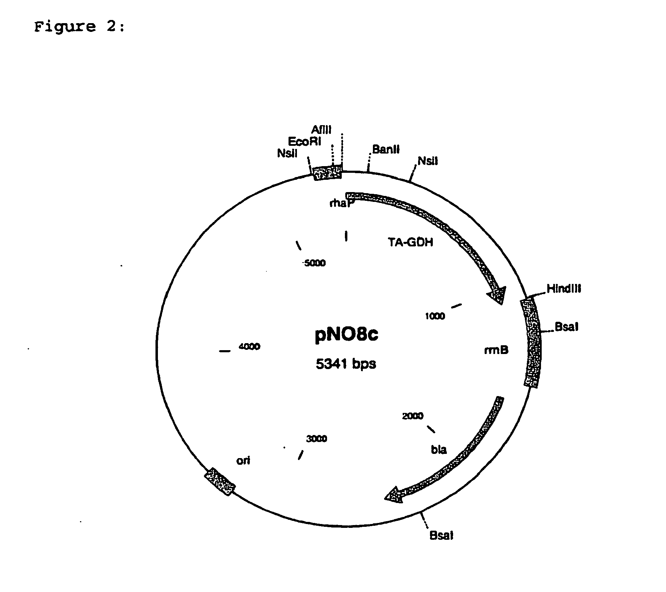

Figure 2 shows the plasmid map of plasmid pNO8c

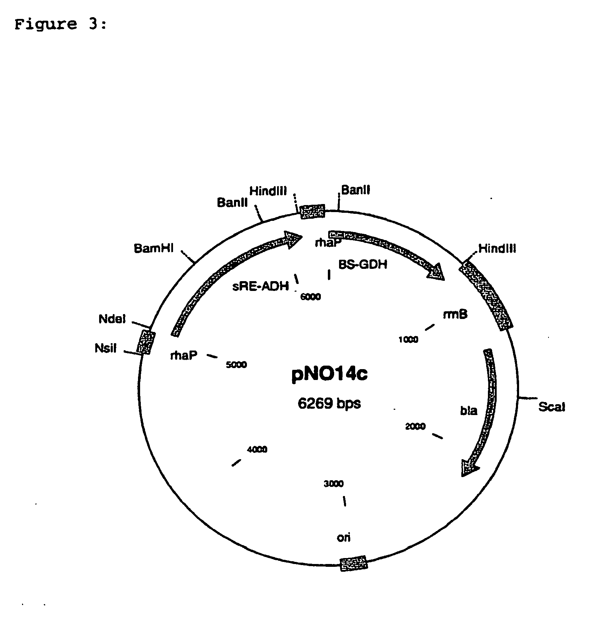

Figure 3 shows the plasmid map of plasmid pNO14c

### Examples:

### Preparation of a whole-cell catalyst comprising an (R)-alcohol dehydrogenase from Lactobacillus kefir and a glucose dehydrogenase from Thermoplasma acidophilum

### Preparation of the strain

Chemically competent cells of *E*. *coli* DSM14459 (described in WO03/042412) were transformed with the plasmid pNO5c (Sambrook et al. 1989, Molecular cloning: A Laboratory Manual, 2nd Edition, Cold Spring Harbor Laboratory Press). This plasmid codes for the alcohol dehydrogenase from *Lactobacillus kefir* (Lactobacillus kefir alcohol dehydrogenase: a useful catalyst for synthesis. Bradshaw et al. JOC 1992, 57 1532-6, Reduction of acetophenone to R(+)-phenylethanol by a new alcohol dehydrogenase from Lactobacillus kefir. Hummel W. Ap Microbiol Biotech 1990, 34, 15-19). The recombinant strain *E. coli* DSM14459 (pNO5c) so prepared was made chemically competent and transformed with the plasmid pNO8c, which codes for the gene of a codon-optimised glucose dehydrogenase from *Thermoplasma acidophilum* (Bright, J.R. et al., 1993 Eur. J. Biochem. 211:549-554). Both genes are under the control of a rhamnose promoter (Stumpp, Tina; Wilms, Burkhard; Altenbuchner, Josef. A new, L-rhamnose-inducible expression system for Escherichia coli. BIOspektrum (2000), 6(1), 33-36). The sequences and plasmid maps of pNO5c and pNO8c are shown hereinbelow.

### Preparation of active cells

An individual colony of *E*. *coli* DSM14459 (pNO5c,pNO8c) was incubated in 2 ml of LB medium with added antibiotic (50 µg/l ampicillin and 20 µg/ml chloramphenicol) for 18 hours at 37°C, with shaking (250 rpm). This culture was diluted 1:100 in fresh LB medium with rhamnose (2 g/l) as inducer, added antibiotic (50 µg/l ampicillin and 20 µg/ml chloramphenicol) and 1 mM ZnCl₂ and was incubated for 18 hours at 30°C, with shaking (250 rpm). The cells were then harvested by centrifugation (10,000 g, 10 min., 4°C), the supernatant was discarded, and the cell pellet was used in biotransformation tests either directly or after storage at -20°C.

### Preparation of a whole-cell catalyst comprising an (S)-alcohol dehydrogenase from Rhodococcus erythropolis and a glucose dehydrogenase from Bacillus subtilis

### Preparation of the strain

Chemically competent cells of *E*. *coli* DSM14459 (described in WO03/042412) were transformed with the plasmid pNO14c (Sambrook et al. 1989, Molecular cloning: A Laboratory Manual, 2nd Edition, Cold Spring Harbor Laboratory Press). This plasmid codes for an alcohol dehydrogenase from *Rhodococcus erythropolis* (Cloning, sequence analysis and heterologous expression of the gene encoding a (S)-specific alcohol dehydrogenase from Rhodococcus erythropolis DSM 43297. Abokitse, K.; Hummel, W. Applied Microbiology and Biotechnology 2003, 62 380-386) and a glucose dehydrogenase from *Bacillus subtilis* (Glucose dehydrogenase from Bacillus subtilis expressed in Escherichia coli. I: Purification, characterization and comparison with glucose dehydrogenase from Bacillus megaterium. Hilt W; Pfleiderer G; Fortnagel P Biochimica et biophysica acta (1991 Jan 29), 1076(2), 298-304). The alcohol dehydrogenase is under the control of a rhamnose promoter (Stumpp, Tina; Wilms, Burkhard; Altenbuchner, Josef. A new, L-rhamnose-inducible expression system for Escherichia coli. BIOspektrum (2000), 6(1), 33-36). The sequence and plasmid map of pNO14c is shown hereinbelow.

### Preparation of active cells

An individual colony of *E*. *coli* DSM14459 (pNO14c) was incubated in 2 ml of LB medium with added antibiotic (50 µg/l ampicillin and 20 µg/ml chloramphenicol) for 18 hours at 37°C, with shaking (250 rpm). This culture was diluted 1:100 in fresh LB medium with rhamnose (2 g/l) as inducer, added antibiotic (50 µg/l ampicillin and 20 µg/ml chloramphenicol) and 1 mM ZnCl₂ and was incubated for 18 hours at 30°C, with shaking (250 rpm). The cells were harvested by centrifugation (10,000 g, 10 min., 4°C), the supernatant was discarded, and the cell pellet was used in biotransformation tests either directly or after storage at -20°C.

### Synthesis Example 1: Reduction of p-chloroacetophenone in a 0.5 M solution using a whole-cell catalyst comprising an (R)-selective alcohol dehydrogenase

In a Titrino reaction vessel there are added to 50 ml of a phosphate buffer (adjusted to pH 7.0) at room temperature the above-described whole-cell catalyst *E. coli* DSM14459 (pNO5c,pNO8c) with an (*R*)-selective alcohol dehydrogenase (*E. coli*, (R)-alcohol dehydrogenase from *L*. *kefir,* glucose dehydrogenase from *T*. *acidophilum*) in a cell concentration of 25 g BWM/l, 1.5 equivalents of glucose (equivalents are based on the amount of *p*-chloroacetophenone used) and 25 mmol. of *p*-chloroacetophenone (corresponding to a substrate concentration, based on phosphate buffer used, of 0.5 M). The reaction mixture is stirred for 7 hours at room temperature, the pH being kept constant by the addition of sodium hydroxide solution (1M NaOH). Samples are taken at regular intervals, and the conversion of the *p*-chloroacetophenone is determined by means of HPLC. After a reaction time of 7 hours, the conversion is >99%.

### Synthesis Example 2: Reduction of p-chloroacetophenone in a 0.5 M solution using a whole-cell catalyst comprising an (S)-selective alcohol dehydrogenase

In a Titrino reaction vessel there are added to 50 ml of a phosphate buffer (adjusted to pH 7.0) at room temperature the above-described whole-cell catalyst *E. coli* DSM14459 (pNO14c) with an (*S*)-selective alcohol dehydrogenase (*E*. *coli,* (S)-alcohol dehydrogenase from *R. erythropolis,* glucose dehydrogenase from *B. subtilis*) in a cell concentration of 50 g BWM/1, 6 equivalents of glucose (equivalents are based on the amount of *p*-chloroacetophenone used) and 25 mmol. of *p*-chloroacetophenone (corresponding to a substrate concentration, based on phosphate buffer used, of 0.5 M). The reaction mixture is stirred for 7.5 hours at room temperature, the pH being kept constant by the addition of sodium hydroxide solution (1M NaOH). Samples are taken at regular intervals, and the conversion of the *p*-chloroacetophenone is determined by means of HPLC. After a reaction time of 7.5 hours, the conversion is 92%.

### Synthesis Example 3: Reduction of acetophenone in a 1.5 M solution using a whole-cell catalyst comprising an (R)-selective alcohol dehydrogenase

In a Titrino reaction vessel there are added to 40 ml of a phosphate buffer (adjusted to pH 7.0) at room temperature the above-described whole-cell catalyst *E. coli* DSM14459 (pNO5c,pNO8c) with an (R)-selective alcohol dehydrogenase (*E. coli,* (R)-alcohol dehydrogenase from *L. kefir,* glucose dehydrogenase from *T*. *acidophilum*) in a cell concentration giving an optical density of OD = 21.15, 1.05 equivalents of glucose (equivalents are based on the amount of acetophenone used) and 60 mmol. of acetophenone (corresponding to a substrate concentration, based on phosphate buffer used, of 1.5 M). The reaction mixture is stirred for 23 hours at room temperature, the pH being kept constant by the addition of sodium hydroxide solution (2M NaOH). Samples are taken at regular intervals, and the conversion of the acetophenone is determined by means of HPLC. After reaction times of 16.5 and 23 hours, the conversion is 93% and 97%, respectively.

## Claims

1. Process for the preparation of optically active alcohols of the general formula 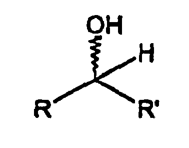 by reduction of the corresponding ketones in the presence of a whole-cell catalyst comprising an alcohol dehydrogenase and also an enzyme capable of cofactor regeneration, **characterised in that** the enzyme capable of co-factor regeneration is glucose dehydrogenase or malate.dehydrogenase and that the conversion of a substrate concentration of at least 500 mM per aqueous solvent used is carried out without the addition of an "external" cofactor, in which the R and R'-radicals are different from one another and are (C₁-C₈)-alkyl, (C₁-C₈)-alkoxy, (C₂-C₈)-alkoxyalkyl, wherein the aforementioned radicals may be mono- or poly-substituted by halogens and/or by radicals containing N, O, P, S, Si atoms, or are (C₃-C₈)-Cycloalkyl, which may be substituted by one or more halogens and/or radicals containing N, O, P, S, Si atoms and/or may contain N, O, P, S atoms in the ring or are HO-(C₁-C₈)-alkgl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl-(C₆-C₁₈)-aryl, (C₁-C₈)-alkyl-(C₃-C₁₈)-heteroaryl, (C₁-C₈)-alkyl-(C₃-C₈)-cycloalkyl, (C₃-C₈)-cycloalkyl-(C₁-C₈)-alkyl, wherein (C₁-C₈) alkyl radical and (C₃-C₈)-cycloalkyl radical have the meaning indicated above.

2. Process according to claim 1 **characterised in that** the concentration of biocatalyst used does not exceed 75 g/l.

3. Process according to claim 1 to 2, **characterised in that** the process is carried out in the absence of an organic solvent.

4. Process according to any one of claims 1 to 3, **characterised in that** there is used a whole-cell catalyst comprising at least one alcohol dehydrogenase selected from the group consisting of an alcohol dehydrogenase from a *Lactobacillus* strain,
, and/or an alcohol dehydrogenase from a *Rhodococcus* strain,
and/or an alcohol dehydrogenase from an *Arthrobacter* strain, .

5. Process according to any one of claims 1 to 4, **characterised in that** the enzyme capable of cofactor regeneration is a glucose dehydrogenase, from *Bacillus, Thermoplasma* and *Pseudomonas* strains.

6. Process according to any one of claims 1 to 5, **characterised in that** the enzyme capable of cofactor regeneration is a malate dehydrogenase.

7. Process according to any one of claims 1 to 6, **characterised in that** the reaction temperature is from 10 to 90°C,

8. Process according to any one of claims 1 to 7, **characterised in that** the pH value is from pH 5 to 9,

9. Process according to any one of claims 1 to 8, **characterised in that** the total amount of substrate is added at the beginning.

10. Process according to any one of claims 1 to 8, **characterised in that** the substrate is metered in during the reaction.

## Patentansprüche

1. Verfahren zur Herstellung optisch aktiver Alkohole der allgemeinen Formel 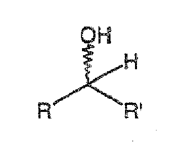 durch Reduktion der entsprechenden Ketone in Gegenwart eines Ganzzellenkatalysators, der eine Alkohol-Dehydrogenase und auch ein zur Cofaktor-Regenerierung fähiges Enzym umfasst, **dadurch gekennzeichnet, dass** es sich bei dem zur Cofaktor-Regenerierung fähigen Enzym um Glucose-Dehydrogenase oder Malat-Dehydrogenase handelt und dass die Umwandlung einer Substratkonzentration von wenigstens 500 mM pro verwendetem wässrigen Lösungsmittel ohne die Zugabe eines "externen" Cofaktors erfolgt, wobei sich die Reste R und R' voneinander unterscheiden und für (C₁-C₈)-Alkyl, (C₁-C₈)-Alkoxy, (C₂-C₈)-Alkoxyalkyl, wobei die vorstehend genannten Reste ein- oder mehrfach mit Halogenen und/oder mit N-, O-, P-, S-, Si-Atome enthaltenden Resten substituiert sein können, oder für (C₃-C₈)-Cycloalkyl, das mit einem oder mehreren Halogenen und/oder N-, O-, P-, S-, Si-Atome enthaltenden Resten substituiert sein und/oder N-, O-, P-, S-Atome im Ring enthalten kann, oder für HO- (C₁-C₈)-Alkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl- (C₆-C₁₈)-aryl, (C₁-C₈)-Alkyl- (C₃-C₁₈)-heteroaryl, (C₁-C₈)-Alkyl-(C₃-C₈)-cycloalkyl, (C₃-C₈)-Cycloalkyl-(C₁-C₈)-alkyl stehen, wobei (C₁-C₈)-Alkylrest und (C₃-C₈)-Cycloalkylrest die oben angegebene Bedeutung aufweisen.

2. Verfahren nach Anspruch 1, **dadurch gekennzeichnet, dass** die Konzentration an verwendetem Biokatalysator nicht mehr als 75 g/l beträgt.

3. Verfahren nach Anspruch 1 bis 2, **dadurch gekennzeichnet, dass** das Verfahren in Abwesenheit eines organischen Lösungsmittels durchgeführt wird.

4. Verfahren nach einem der Ansprüche 1 bis 3, **dadurch gekennzeichnet, dass** dabei ein Ganzzellenkatalysator verwendet wird, der wenigstens eine Alkohol-Dehydrogenase, ausgewählt aus der Gruppe bestehend aus einer Alkohol-Dehydrogenase aus einem *Lactobacillus-*Stamm und/oder einer Alkohol-Dehydrogenase aus einem Rhodococcus-Stamm und/oder einer Alkohol-Dehydrogenase aus einem *Arthrobacter*-Stamm, umfasst.

5. Verfahren nach einem der Ansprüche 1 bis 4, **dadurch gekennzeichnet, dass** es sich bei dem zur Cofaktor-Regenerierung fähigen Enzym um eine Glucose-Dehydrogenase handelt, und zwar aus *Bacillus-, Thermoplasma- und Pseudomonas*-Stämmen.

6. Verfahren nach einem der Ansprüche 1 bis 5, **dadurch gekennzeichnet, dass** es sich bei dem zur Cofaktor-Regenerierung fähigen Enzym um eine Malat-Dehydrogenase handelt.

7. Verfahren nach einem der Ansprüche 1 bis 6, **dadurch gekennzeichnet, dass** die Reaktionstemperatur 10 bis 90°C beträgt.

8. Verfahren nach einem der Ansprüche 1 bis 7, **dadurch gekennzeichnet, dass** der pH-Wert im Bereich von pH 5 bis pH 9 liegt.

9. Verfahren nach einem der Ansprüche 1 bis 8, **dadurch gekennzeichnet, dass** die gesamte Menge an Substrat zu Beginn zugegeben wird.

10. Verfahren nach einem der Ansprüche 1 bis 8, **dadurch gekennzeichnet, dass** das Substrat während der Reaktion zudosiert wird.

## Revendications

1. Procédé pour la préparation d'alcools optiquement actifs de formule générale 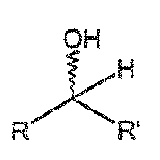 par réduction des cétones correspondantes en présence d'un catalyseur de cellules entières comprenant une alcool déshydrogénase et également une enzyme capable de régénération de cofacteur, **caractérisé en ce que** l'enzyme capable de régénération de cofacteur est la glucose déshydrogénase ou la malate déshydrogénase et que la conversion d'une concentration de substrat d'au moins 500 mM par rapport au solvant aqueux utilisé est effectuée sans l'ajout d'un cofacteur « externe » où les radicaux R et R' sont différents l'un de l'autre et sont un alkyle en C₁-C₈, un alcoxy en C₁-C₈, un alcoxyalkyle en C₂-C₈, où les radicaux mentionnés ci-dessus peuvent être mono- ou poly-substitués par des halogènes et/ou par des radicaux contenant des atomes N, 0, P, S, Si, ou sont un cycloalkyle en C₃-C₈, qui peut être substitué par un ou plusieurs halogènes et/ou radicaux contenant des atomes N, 0, P, S, Si et/ou peut contenir des atomes N, 0, P, S dans le cycle ou sont HO-(alkyle en C₁-C₈), un aryle en C₆-C₁₈, un aralkyle en C₇-C₁₉, un hétéroaryle en C₃-C₁₈, un hétéroaralkyle en C₄-C₁₉, un (alkyle en C₁-C₈)-(aryle en C₆-C₁₈), un (alkyle en C₁-C₈)-(hétéroaryle en C₃-C₁₈), un (alkyle en C₁-C₈)-(cycloalkyle en C₃-C₈), un (cycloalkyle en C₃-C₈)-(alkyle en C₁-C₈), où le radical alkyle en C₁-C₈ et le radical cycloalkyle en C₃-C₈ ont la définition ci-dessus.

2. Procédé selon la revendication 1 **caractérisé en ce que** la concentration de biocatalyseur utilisée ne dépasse pas 75 g/l.

3. Procédé selon la revendication 1 à 2, **caractérisé en ce que** le procédé est conduit en l'absence d'un solvant organique.

4. Procédé selon l'une quelconque des revendications 1 à 3, **caractérisé en ce qu'**il est utilisé un catalyseur de cellules entières comprenant au moins une alcool déshydrogénase choisie dans le groupe constitué d'une alcool déshydrogénase d'une souche de *Lactobacillus,* et/ou une alcool déshydrogénase d'une souche de *Rhodococcus,* et/ou une alcool déshydrogénase d'une souche d'*Arthrobacter*.

5. Procédé selon l'une quelconque des revendications 1 à 4, **caractérisé en ce que** l'enzyme capable de régénération de cofacteur est une glucose déshydrogénase, des souches *Thermoplasma* et *Pseudomonas* de *Bacillus.*

6. Procédé selon l'une quelconque des revendications 1 à 5, **caractérisé en ce que** l'enzyme capable de régénération de cofacteur est une malate déshydrogénase.

7. Procédé selon l'une quelconque des revendications 1 à 6, **caractérisé en ce que** la température de réaction est de 10 à 90 °C.

8. Procédé selon l'une quelconque des revendications 1 à 7, **caractérisé en ce que** la valeur de pH est de pH 5 à 9.

9. Procédé selon l'une quelconque des revendications 1 à 8, **caractérisé en ce que** la quantité totale de substrat est ajoutée au début.

10. Procédé selon l'une quelconque des revendications 1 à 8, **caractérisé en ce que** le substrat est ajouté de manière dosée pendant la réaction.


### SEQUENCE LISTING

<110> Degussa AG
<120> Whole-cell reduction of ketones <130> 040175 OC
<160> 3
<170> PatentIn version 3.1
<210> 1
   <211> 5740
   <212> DNA
   <213> pNO5c with Lactobacillus kefir -ADH
<400> 1
   aattcttaag aaggagatat acatatgact gatcgtttaa aaggcaaagt agcaattgta 60
   actggcggta ccttgggaat tggcttggca atcgctgata agtttgttga agaaggcgca 120
   aaggttgtta ttaccggccg tcacgctgat gtaggtgaaa aagctgccaa atcaatcggc 180
   ggcacagacg ttatccgttt tgtccaacac gatgcttctg atgaagccgg ctggactaag 240
   ttgtttgata cgactgaaga agcatttggc ccagttacca cggttgtcaa caatgccgga 300
   attgcggtca gcaagagtgt tgaagatacc acaactgaag aatggcgcaa gctgctctca 360
   gttaacttgg atggtgtctt cttcggtacc cgtcttggaa tccaacgtat gaagaataaa 420
   ggactcggag catcaatcat caatatgtca tctatcgaag gttttgttgg tgatccaact 480
   ctgggtgcat acaacgcttc aaaaggtgct gtcagaatta tgtctaaatc agctgccttg 540
   gattgcgctt tgaaggacta cgatgttcgg gttaacactg ttcatccagg ttatatcaag 600
   acaccattgg ttgacgatct tgaaggggca gaagaaatga tgtcacagcg gaccaagaca 660
   ccaatgggtc atatcggtga acctaacgat atcgcttgga tctgtgttta cctggcatct 720
   gacgaatcta aatttgccac tggtgcagaa ttcgttgtcg atggtggata cactgctcaa 780
   taaggatccg aattcgagct caggaggtat catatgcagc caagcttctg ttttggcgga 840
   tgagagaaga ttttcagcct gatacagatt aaatcagaac gcagaagcgg tctgataaaa 900
   cagaatttgc ctggcggcag tagcgcggtg gtcccacctg accccatgcc gaactcagaa 960
   gtgaaacgcc gtagcgccga tggtagtgtg gggtctcccc atgcgagagt agggaactgc 1020
   caggcatcaa ataaaacgaa aggctcagtc gaaagactgg gcctttcgtt ttatctgttg 1080
   tttgtcggtg aacgctctcc tgagtaggac aaatccgccg ggagcggatt tgaacgttgc 1140
   gaagcaacgg cccggagggt ggcgggcagg acgcccgcca taaactgcca ggcatcaaat 1200
   taagcagaag gccatcctga cggatggcct ttttgcgttt ctacaaactc ttttgtttat 1260
   ttttctaaat acattcaaat atgtatccgc tcatgagaca ataaccctga taaatgcttc 1320
   aataatatcg tccattccga cagcatcgcc agtcactatg gcgtgctgct agcgctatat 1380
   gcgttgatgc aatttctatg cgcacccgtt ctcggagcac tgtccgaccg ctttggccgc 1440
   cgcccagtcc tgctcgcttc gctacttgga gccactatcg actacgcgat catggcgacc   1500
   acacccgtcc tgtggatcct ctacgccgga cgcatcgtgg ccggcatcac cggcgccaca   1560
   ggtgcggttg ctggcgccta tatcgccgac atcaccgatg gggaagatcg ggctcgccac   1620
   ttcgggctca tgagcgcttg tttcggcgtg ggtatggtgg caggccccgt ggccggggga   1680
   ctgttgggcg ccatctcctt gcatgcacca ttccttgcgg cggcggtgct caacggcctc   1740
   aacctactac tgggctgctt cctaatgcag gagtcgcata agggagagcg tcgaccgatg   1800
   cccttgagag ccttcaaccc agtcagctcc ttccggtggg cgcggggcat gactatcgtc   1860
   gccgcactta tgactgtctt ctttatcatg caactcgtag gacaggtgcc ggcagcgctc   1920
   tgggtcattt tcggcgagga ccgctttcgc tggagcgcga cgatgatcgg cctgtcgctt   1980
   gcggtattcg gaatcttgca cgccctcgct caagccttcg tcactggtcc cgccaccaaa   2040
   cgtttcggcg agaagcaggc cattatcgcc ggcatggcgg ccgacgcgct gggctacgtc   2100
   ttgctggcgt tcgcgacgcg aggctggatg gccttcccca ttatgattct tctcgcttcc   2160
   ggcggcatcg ggatgcccgc gttgcaggcc atgctgtcca ggcaggtaga tgacgaccat   2220
   cagggacagc ttcaaggatc gctcgcggct cttaccagcc taacttcgat cactggaccg   2280
   ctgatcgtca cggcgattta tgccgcctcg gcgagcacat ggaacgggtt ggcatggatt   2340
   gtaggcgccg ccctatacct tgtctgcctc cccgcgttgc gtcgcggtgc atggagccgg   2400
   gccacctcga cctgaatgga agccggcggc acctcgctaa cggattcacc actccaagaa   2460
   ttggagccaa tcaattcttg cggagaactg tgaatgcgca aaccaaccct tggcagaaca   2520
   tatccatcgc gtccgccatc tccagcagcc gcacgcggcg catctcgggc agcgttgggt   2580
   cctggccacg ggtgcgcatg atcgtgctcc tgtcgttgag gacccggcta ggctggcggg   2640
   gttgccttac tggttagcag aatgaatcac cgatacgcga gcgaacgtga agcgactgct   2700
   gctgcaaaac gtctgcgacc tgagcaacaa catgaatggt cttcggtttc cgtgtttcgt   2760
   aaagtctgga aacgcggaag tcccctacgt gctgctgaag ttgcccgcaa cagagagtgg   2820
   aaccaaccgg tgataccacg atactatgac tgagagtcaa cgccatgagc ggcctcattt   2880
   cttattctga gttacaacag tccgcaccgc tgtccggtag ctccttccgg tgggcgcggg   2940
   gcatgactat cgtcgccgca cttatgactg tcttctttat catgcaactc gtaggacagg   3000
   tgccggcagc gcccaacagt cccccggcca cggggcctgc caccataccc acgccgaaac   3060
   aagcgccctg caccattatg ttccggatct gcatcgcagg atgctgctgg ctaccctgtg   3120
   gaacacctac atctgtatta acgaagcgct aaccgttttt atcaggctct gggaggcaga   3180
   ataaatgatc atatcgtcaa ttattacctc cacggggaga gcctgagcaa actggcctca   3240
   ggcatttgag aagcacacgg tcacactgct tccggtagtc aataaaccgg taaaccagca   3300
   atagacataa gcggctattt aacgaccctg ccctgaaccg acgaccgggt cgaatttgct   3360
   ttcgaatttc tgccattcat ccgcttatta tcacttattc aggcgtagca ccaggcgttt 3420
   aagggcacca ataactgcct taaaaaaatt acgccccgcc ctgccactca tcgcagtact 3480
   gttgtaattc attaagcatt ctgccgacat ggaagccatc acagacggca tgatgaacct 3540
   gaatcgccag cggcatcagc accttgtcgc cttgcgtata atatttgccc atggtgaaaa 3600
   cgggggcgaa gaagttgtcc atattggcca cgtttaaatc aaaactggtg aaactcaccc 3660
   agggattggc tgagacgaaa aacatattct caataaaccc tttagggaaa taggccaggt 3720
   tttcaccgta acacgccaca tcttgcgaat atatgtgtag aaactgccgg aaatcgtcgt 3780
   ggtattcact ccagagcgat gaaaacgttt cagtttgctc atggaaaacg gtgtaacaag 3840
   ggtgaacact atcccatatc accagctcac cgtctttcat tgccatacga attccggatg 3900
   agcattcatc aggcgggcaa gaatgtgaat aaaggccgga taaaacttgt gcttattttt 3960
   ctttacggtc tttaaaaagg ccgtaatatc cagctgaacg gtctggttat aggtacattg 4020
   agcaactgac tgaaatgcct caaaatgttc tttacgatgc cattgggata tatcaacggt 4080
   ggtatatcca gtgatttttt tctccatttt agcttcctta gctcctgaaa atctcgataa 4140
   ctcaaaaaat acgcccggta gtgatcttat ttcattatgg tgaaagttgg aacctcttac 4200
   gtgccgatca acgtctcatt ttcgccaaaa gttggcccag ggcttcccgg tatcaacagg 4260
   gacaccagga tttatttatt ctgcgaagtg atcttccgtc acaggtattt attcggcgca 4320
   aagtgcgtcg ggtgatgctg ccaacttact gatttagtgt atgatggtgt ttttgaggtg 4380
   ctccagtggc ttctgtttct atcagctgtc cctcctgttc agctactgac ggggtggtgc 4440
   gtaacggcaa aagcaccgcc ggacatcagc gctagcggag tgtatactgg cttactatgt 4500
   tggcactgat gagggtgtca gtgaagtgct tcatgtggca ggagaaaaaa ggctgcaccg 4560
   gtgcgtcagc agaatatgtg atacaggata tattccgctt cctcgctcac tgactcgcta 4620
   cgctcggtcg ttcgactgcg gcgagcggaa atggcttacg aacggggcgg agatttcctg 4680
   gaagatgcca ggaagatact taacagggaa gtgagagggc cgcggcaaag ccgtttttcc 4740
   ataggctccg cccccctgac aagcatcacg aaatctgacg ctcaaatcag tggtggcgaa 4800
   acccgacagg actataaaga taccaggcgt ttcccctggc ggctccctcg tgcgctctcc 4860
   tgttcctgcc tttcggttta ccggtgtcat tccgctgtta tggccgcgtt tgtctcattc 4920
   cacgcctgac actcagttcc gggtaggcag ttcgctccaa gctggactgt atgcacgaac 4980
   cccccgttca gtccgaccgc tgcgccttat ccggtaacta tcgtcttgag tccaacccgg 5040
   aaagacatgc aaaagcacca ctggcagcag ccactggtaa ttgatttaga ggagttagtc 5100
   ttgaagtcat gcgccggtta aggctaaact gaaaggacaa gttttggtga ctgcgctcct 5160
   ccaagccagt tacctcggtt caaagagttg gtagctcaga gaaccttcga aaaaccgccc 5220
   tgcaaggcgg ttttttcgtt ttcagagcaa gagattacgc gcagaccaaa acgatctcaa 5280
   gaagatcatc ttattaagct ttaatgcggt agtttatcac agttaaattg ctaacgcagt 5340
   caggcaccgt gtatgaaatc taacaatgcg ctcatcgtca tcctcggcac cgtcaccctg 5400
   gatgctgtag gcataggctt ggttatgccg gtactgccgg gcctcttgcg ggattagtca 5460
   tgccccgcgc ccaccggaag gagctgactg ggttgaaggc tctcaagggc atcggtcgac 5520
   gctctccctt atgcgactcc tgcattagga agcagcccag tagtaggttg aggccgttga 5580
   gcaccgccgc cgcaaggaat ggtgcatgca tcgatcacca caattcagca aattgtgaac 5640
   atcatcacgt tcatctttcc ctggttgcca atggcccatt ttcctgtcag taacgagaag 5700
   gtcgcgaatt caggcgcttt ttagactggt cgtaatgaac 5740
<210> 2
   <211> 5341
   <212> DNA
   <213> pNO8c with Thermoplasma acidophilum - GDH
<400> 2
   tatgaccgag cagaaagcga ttgtgaccga tgcgccgaaa ggtggtgtga aatacaccac 60
   cattgatatg ccggaaccgg aacattatga tgcgaaactg agcccggtgt atatcggtat 120
   ttgcggcacc gatcgtggtg aagtggcggg tgcgctgagc tttacctata acccggaagg 180
   cgaaaacttt ctggtgctgg gccatgaagc gctgctgcgt gtggatgatg cgcgtgataa 240
   cggctatatc aaaaagggcg atctggtggt gccgctggtg cgtcgtccgg gtaaatgcat 300
   taactgccgc attggccgtc aggataactg tagcattggc gatccggata aacatgaagc 360
   gggcattacc ggcctgcatg gctttatgcg cgatgtgatc tatgatgata ttgaatatct 420
   ggtgaaagtg gaagatccgg aactgggtcg tattgcggtg ctgaccgaac cgctgaaaaa 480
   cgtgatgaaa gcgtttgaag tgtttgatgt ggtgagcaaa tgcagcattt tctttggcga 540
   tgatagcacc ctgattggca aacgcatggt gattatcggc agcggtagcg aagcgtttct 600
   gtatagcttt gcgggcgtgg atcgtggttt tgatgtgacc atggtgaacc gccatgatga 660
   aaccgaaaac aaactgaaaa tcatggatga atttggcgtg aaattcgcga actatctgaa 720
   agatatgccg gagaaaatcg atctgctggt tgataccagc ggtgatccga ccaccacctt 780
   caaattcctg cgcaaagtga acaacaacgg cgtggtgatt ctgtttggca ccaacggtaa 840
   agcgccgggt tatccggtgg atggcgaaga tattgattac attgtggaac gcaacattac 900
   cattgcgggt agcgtggatg cggcgaaaat ccattatgtg caggcgctgc aaagcctgag 960
   caactggaat cgtcgtcatc cggatgcgat gaaaagcatt atcacctatg aagcgaaacc 1020
   gagcgaaacc aacattttct ttcagaaacc gcatggcgaa atcaaaaccg tgatcaaatg 1080
   gcagtaagct tctgttttgg cggatgagag aagattttca gcctgataca gattaaatca 1140
   gaacgcagaa gcggtctgat aaaacagaat ttgcctggcg gcagtagcgc ggtggtccca 1200
   cctgacccca tgccgaactc agaagtgaaa cgccgtagcg ccgatggtag tgtggggtct 1260
   ccccatgcga gagtagggaa ctgccaggca tcaaataaaa cgaaaggctc agtcgaaaga 1320
   ctgggccttt cgttttatct gttgtttgtc ggtgaacgct ctcctgagta ggacaaatcc 1380
   gccgggagcg gatttgaacg ttgcgaagca acggcccgga gggtggcggg caggacgccc 1440
   gccataaact gccaggcatc aaattaagca gaaggccatc ctgacggatg gcctttttgc 1500
   gtttctacaa actcttttgt ttatttttct aaatacattc aaatatgtat ccgctcatga 1560
   gacaataacc ctgataaatg cttcaataat attgaaaaag gaagagtatg agtattcaac 1620
   atttccgtgt cgcccttatt cccttttttg cggcattttg ccttcctgtt tttgctcacc 1680
   cagaaacgct ggtgaaagta aaagatgctg aagatcagtt gggtgcacga gtgggttaca 1740
   tcgaactgga tctcaacagc ggtaagatcc ttgagagttt tcgccccgaa gaacgttttc 1800
   caatgatgag cacttttaaa gttctgctat gtggcgcggt attatcccgt gttgacgccg 1860
   ggcaagagca actcggtcgc cgcatacact attctcagaa tgacttggtt gagtactcac 1920
   cagtcacaga aaagcatctt acggatggca tgacagtaag agaattatgc agtgctgcca 1980
   taaccatgag tgataacact gcggccaact tacttctgac aacgatcgga ggaccgaagg 2040
   agctaaccgc ttttttgcac aacatggggg atcatgtaac tcgccttgat cgttgggaac 2100
   cggagctgaa tgaagccata ccaaacgacg agcgtgacac cacgatgcct gtagcaatgg 2160
   caacaacgtt gcgcaaacta ttaactggcg aactacttac tctagcttcc cggcaacaat 2220
   taatagactg gatggaggcg gataaagttg caggaccact tctgcgctcg gcccttccgg 2280
   ctggctggtt tattgctgat aaatctggag ccggtgagcg tgggtctcgc ggtatcattg 2340
   cagcactggg gccagatggt aagccctccc gtatcgtagt tatctacacg acggggagtc 2400
   aggcaactat ggatgaacga aatagacaga tcgctgagat aggtgcctca ctgattaagc 2460
   attggtaact gtcagaccaa gtttactcat atatacttta gattgattta aaacttcatt 2520
   tttaatttaa aaggatctag gtgaagatcc tttttgataa tctcatgacc aaaatccctt 2580
   aacgtgagtt ttcgttccac tgagcgtcag accccgtaga aaagatcaaa ggatcttctt 2640
   gagatccttt ttttctgcgc gtaatctgct gcttgcaaac aaaaaaacca ccgctaccag 2700
   cggtggtttg tttgccggat caagagctac caactctttt tccgaaggta actggcttca 2760
   gcagagcgca gataccaaat actgtccttc tagtgtagcc gtagttaggc caccacttca 2820
   agaactctgt agcaccgcct acatacctcg ctctgctaat cctgttacca gtggctgctg 2880
   ccagtggcga taagtcgtgt cttaccgggt tggactcaag acgatagtta ccggataagg 2940
   cgcagcggtc gggctgaacg gggggttcgt gcacacagcc cagcttggag cgaacgacct 3000
   acaccgaact gagataccta cagcgtgagc tatgagaaag cgccacgctt cccgaaggga 3060
   gaaaggcgga caggtatccg gtaagcggca gggtcggaac aggagagcgc acgagggagc 3120
   ttccaggggg aaacgcctgg tatctttata gtcctgtcgg gtttcgccac ctctgacttg 3180
   agcgtcgatt tttgtgatgc tcgtcagggg ggcggagcct atggaaaaac gccagcaacg 3240
   cggccttttt acggttcctg gccttttgct ggccttttgc tcacatgttc tttcctgcgt 3300
   tatcccctga ttctgtggat aaccgtatta ccgcctttga gtgagctgat accgctcgcc 3360
   gcagccgaac gaccgagcgc agcgagtcag tgagcgagga agcggaagag cgcctgatgc 3420
   ggtattttct ccttacgcat ctgtgcggta tttcacaccg catatatggt gcactctcag 3480
   tacaatctgc tctgatgccg catagttaag ccagtataca ctccgctatc gctacgtgac 3540
   tgggtcatgg ctgcgccccg acacccgcca acacccgctg acgcgccctg acgggcttgt 3600
   ctgctcccgg catccgctta cagacaagct gtgaccgtct ccgggagctg catgtgtcag 3660
   aggttttcac cgtcatcacc gaaacgcgcg aggcagctgc ggtaaagctc atcagcgtgg 3720
   tcgtgaagcg attcacagat gtctgcctgt tcatccgcgt ccagctcgtt gagtttctcc 3780
   agaagcgtta atgtctggct tctgataaag cgggccatgt taagggcggt tttttcctgt 3840
   ttggtcactt gatgcctccg tgtaaggggg aatttctgtt catgggggta atgataccga 3900
   tgaaacgaga gaggatgctc acgatacggg ttactgatga tgaacatgcc cggttactgg 3960
   aacgttgtga gggtaaacaa ctggcggtat ggatgcggcg ggaccagaga aaaatcactc 4020
   agggtcaatg ccagcgcttc gttaatacag atgtaggtgt tccacagggt agccagcagc 4080
   atcctgcgat gcagatccgg aacataatgg tgcagggcgc tgacttccgc gtttccagac 4140
   tttacgaaac acggaaaccg aagaccattc atgttgttgc tcaggtcgca gacgttttgc 4200
   agcagcagtc gcttcacgtt cgctcgcgta tcggtgattc attctgctaa ccagtaaggc 4260
   aaccccgcca gcctagccgg gtcctcaacg acaggagcac gatcatgcgc acccgtggcc 4320
   aggacccaac gctgcccgag atgcgccgcg tgcggctgct ggagatggcg gacgcgatgg 4380
   atatgttctg ccaagggttg gtttgcgcat tcacagttct ccgcaagaat tgattggctc 4440
   caattcttgg agtggtgaat ccgttagcga ggtgccgccg gcttccattc aggtcgaggt 4500
   ggcccggctc catgcaccgc gacgcaacgc ggggaggcag acaaggtata ccatgccaac 4560
   ccgttccatg tgctcgccga ggcggcataa atcgccgtga cgatcagcgg tccagtgatc 4620
   gaagttaggc tggtaagagc cgcgagcgat ccttgaagct gtccctgatg gtcgtcatct 4680
   acctgcctgg acagcatggc ctgcaacgcg ggcatcccga tgccgccgga agcgagaaga 4740
   atcataatgg ggaaggccat ccagcctcgc gtcgcgaacg ccagcaagac gtagcccagc 4800
   gcgtcggccg ccatgccggc gataatggcc tgcttctcgc cgaaacgttt ggtggcggga 4860
   ccagtgacga aggcttgagc gagggcgtgc aagattccga ataccgcaag cgacaggccg 4920
   atcatcgtcg cgctccagcg aaagcggtcc tcgccgaaaa tgacccagag cgctgccggc 4980
   acctgtccta cgagttgcat gataaagaag acagtcataa gtgcggcgac gatagtcatg 5040
   ccccgcgccc accggaagga gctgactggg ttgaaggctc tcaagggcat cggtcgacgc 5100
   tctcccttat gcgactcctg cattaggaag cagcccagta gtaggttgag gccgttgagc 5160
   accgccgccg caaggaatgg tgcatgcatc gatcaccaca attcagcaaa ttgtgaacat 5220
   catcacgttc atctttccct ggttgccaat ggcccatttt cctgtcagta acgagaaggt 5280
   cgcgaattca ggcgcttttt agactggtcg taatgaacaa ttcttaagaa ggagatatac 5340
   a 5341
<210> 3
   <211> 6269
   <212> DNA
   <213> pNO14c with R. erythropolis - sADH and B. subtilis - GDH
<400> 3
   tatgtatccg gatttaaaag gaaaagtcgt cgctattaca ggagctgctt cagggctcgg 60
   aaaggcgatg gccattcgct tcggcaagga gcaggcaaaa gtggttatca actattatag 120
   taataaacaa gatccgaacg aggtaaaaga agaggtcatc aaggcgggcg gtgaagctgt 180
   tgtcgtccaa ggagatgtca cgaaagagga agatgtaaaa aatatcgtgc aaacggcaat 240
   taaggagttc ggcacactcg atattatgat taataatgcc ggtcttgaaa atcctgtgcc 300
   atctcacgaa atgccgctca aggattggga taaagtcatc ggcacgaact taacgggtgc 360
   ctttttagga agccgtgaag cgattaaata tttcgtagaa aacgatatca agggaaatgt 420
   cattaacatg tccagtgtgc acgaagtgat tccttggccg ttatttgtcc actatgcggc 480
   aagtaaaggc gggataaagc tgatgacaga aacattagcg ttggaatacg cgccgaaggg 540
   cattcgcgtc aataatattg ggccaggtgc gatcaacacg ccaatcaatg ctgaaaaatt 600
   cgctgaccct aaacagaaag ctgatgtaga aagcatgatt ccaatgggat atatcggcga 660
   accggaggag atcgccgcag tagcagcctg gcttgcttcg aaggaagcca gctacgtcac 720
   aggcatcacg ttattcgcgg acggcggtat gacacaatat ccttcattcc aggcaggccg 780
   cggttaatag tagaagcttc tgttttggcg gatgagagaa gattttcagc ctgatacaga 840
   ttaaatcaga acgcagaagc ggtctgataa aacagaattt gcctggcggc agtagcgcgg 900
   tggtcccacc tgaccccatg ccgaactcag aagtgaaacg ccgtagcgcc gatggtagtg 960
   tggggtctcc ccatgcgaga gtagggaact gccaggcatc aaataaaacg aaaggctcag 1020
   tcgaaagact gggcctttcg ttttatctgt tgtttgtcgg tgaacgctct cctgagtagg 1080
   acaaatccgc cgggagcgga tttgaacgtt gcgaagcaac ggcccggagg gtggcgggca 1140
   ggacgcccgc cataaactgc caggcatcaa attaagcaga aggccatcct gacggatggc 1200
   ctttttgcgt ttctacaaac tcttttgttt atttttctaa atacattcaa atatgtatcc 1260
   gctcatgaga caataaccct gataaatgct tcaataatat tgaaaaagga agagtatgag 1320
   tattcaacat ttccgtgtcg cccttattcc cttttttgcg gcattttgcc ttcctgtttt 1380
   tgctcaccca gaaacgctgg tgaaagtaaa agatgctgaa gatcagttgg gtgcacgagt 1440
   gggttacatc gaactggatc tcaacagcgg taagatcctt gagagttttc gccccgaaga 1500
   acgttttcca atgatgagca cttttaaagt tctgctatgt ggcgcggtat tatcccgtgt 1560
   tgacgccggg caagagcaac tcggtcgccg catacactat tctcagaatg acttggttga 1620
   gtactcacca gtcacagaaa agcatcttac ggatggcatg acagtaagag aattatgcag 1680
   tgctgccata accatgagtg ataacactgc ggccaactta cttctgacaa cgatcggagg 1740
   accgaaggag ctaaccgctt ttttgcacaa catgggggat catgtaactc gccttgatcg 1800
   ttgggaaccg gagctgaatg aagccatacc aaacgacgag cgtgacacca cgatgcctgt 1860
   agcaatggca acaacgttgc gcaaactatt aactggcgaa ctacttactc tagcttcccg 1920
   gcaacaatta atagactgga tggaggcgga taaagttgca ggaccacttc tgcgctcggc 1980
   ccttccggct ggctggttta ttgctgataa atctggagcc ggtgagcgtg ggtctcgcgg 2040
   tatcattgca gcactggggc cagatggtaa gccctcccgt atcgtagtta tctacacgac 2100
   ggggagtcag gcaactatgg atgaacgaaa tagacagatc gctgagatag gtgcctcact 2160
   gattaagcat tggtaactgt cagaccaagt ttactcatat atactttaga ttgatttaaa 2220
   acttcatttt taatttaaaa ggatctaggt gaagatcctt tttgataatc tcatgaccaa 2280
   aatcccttaa cgtgagtttt cgttccactg agcgtcagac cccgtagaaa agatcaaagg 2340
   atcttcttga gatccttttt ttctgcgcgt aatctgctgc ttgcaaacaa aaaaaccacc 2400
   gctaccagcg gtggtttgtt tgccggatca agagctacca actctttttc cgaaggtaac 2460
   tggcttcagc agagcgcaga taccaaatac tgtccttcta gtgtagccgt agttaggcca 2520
   ccacttcaag aactctgtag caccgcctac atacctcgct ctgctaatcc tgttaccagt 2580
   ggctgctgcc agtggcgata agtcgtgtct taccgggttg gactcaagac gatagttacc 2640
   ggataaggcg cagcggtcgg gctgaacggg gggttcgtgc acacagccca gcttggagcg 2700
   aacgacctac accgaactga gatacctaca gcgtgagcta tgagaaagcg ccacgcttcc 2760
   cgaagggaga aaggcggaca ggtatccggt aagcggcagg gtcggaacag gagagcgcac 2820
   gagggagctt ccagggggaa acgcctggta tctttatagt cctgtcgggt ttcgccacct 2880
   ctgacttgag cgtcgatttt tgtgatgctc gtcagggggg cggagcctat ggaaaaacgc 2940
   cagcaacgcg gcctttttac ggttcctggc cttttgctgg ccttttgctc acatgttctt 3000
   tcctgcgtta tcccctgatt ctgtggataa ccgtattacc gcctttgagt gagctgatac 3060
   cgctcgccgc agccgaacga ccgagcgcag cgagtcagtg agcgaggaag cggaagagcg 3120
   cctgatgcgg tattttctcc ttacgcatct gtgcggtatt tcacaccgca tatatggtgc 3180
   actctcagta caatctgctc tgatgccgca tagttaagcc agtatacact ccgctatcgc 3240
   tacgtgactg ggtcatggct gcgccccgac acccgccaac acccgctgac gcgccctgac 3300
   gggcttgtct gctcccggca tccgcttaca gacaagctgt gaccgtctcc gggagctgca 3360
   tgtgtcagag gttttcaccg tcatcaccga aacgcgcgag gcagctgcgg taaagctcat 3420
   cagcgtggtc gtgaagcgat tcacagatgt ctgcctgttc atccgcgtcc agctcgttga 3480
   gtttctccag aagcgttaat gtctggcttc tgataaagcg ggccatgtta agggcggttt 3540
   tttcctgttt ggtcacttga tgcctccgtg taagggggaa tttctgttca tgggggtaat 3600
   gataccgatg aaacgagaga ggatgctcac gatacgggtt actgatgatg aacatgcccg 3660
   gttactggaa cgttgtgagg gtaaacaact ggcggtatgg atgcggcggg accagagaaa 3720
   aatcactcag ggtcaatgcc agcgcttcgt taatacagat gtaggtgttc cacagggtag 3780
   ccagcagcat cctgcgatgc agatccggaa cataatggtg cagggcgctg acttccgcgt 3840
   ttccagactt tacgaaacac ggaaaccgaa gaccattcat gttgttgctc aggtcgcaga 3900
   cgttttgcag cagcagtcgc ttcacgttcg ctcgcgtatc ggtgattcat tctgctaacc 3960
   agtaaggcaa ccccgccagc ctagccgggt cctcaacgac aggagcacga tcatgcgcac 4020
   ccgtggccag gacccaacgc tgcccgagat gcgccgcgtg cggctgctgg agatggcgga 4080
   cgcgatggat atgttctgcc aagggttggt ttgcgcattc acagttctcc gcaagaattg 4140
   attggctcca attcttggag tggtgaatcc gttagcgagg tgccgccggc ttccattcag 4200
   gtcgaggtgg cccggctcca tgcaccgcga cgcaacgcgg ggaggcagac aaggtatagg 4260
   cctacaatcc atgccaaccc gttccatgtg ctcgccgagg cggcataaat cgccgtgacg 4320
   atcagcggtc cagtgatcga agttaggctg gtaagagccg cgagcgatcc ttgaagctgt 4380
   ccctgatggt cgtcatctac ctgcctggac agcatggcct gcaacgcggg catcccgatg 4440
   ccgccggaag cgagaagaat cataatgggg aaggccatcc agcctcgcgt cgcgaacgcc 4500
   agcaagacgt agcccagcgc gtcggccgcc atgccggcga taatggcctg cttctcgccg 4560
   aaacgtttgg tggcgggacc agtgacgaag gcttgagcga gggcgtgcaa gattccgaat 4620
   accgcaagcg acaggccgat catcgtcgcg ctccagcgaa agcggtcctc gccgaaaatg 4680
   acccagagcg ctgccggcac ctgtcctacg agttgcatga taaagaagac agtcataagt 4740
   gcggcgacga tagtcatgcc ccgcgcccac cggaaggagc tgactgggtt gaaggctctc 4800
   aagggcatcg gtcgacgctc tcccttatgc gactcctgca ttaggaagca gcccagtagt 4860
   aggttgaggc cgttgagcac cgccgccgca aggaatggtg catgcatcga tcaccacaat 4920
   tcagcaaatt gtgaacatca tcacgttcat ctttccctgg ttgccaatgg cccattttcc 4980
   tgtcagtaac gagaaggtcg cgaattcagg cgctttttag actggtcgta atgaacaatt 5040
   cttaagaagg agatatacat atgaaagcga tccagtacac ccgtattggt gcggaaccgg 5100
   aactgaccga aatcccgaaa ccggaaccgg gtccgggtga agttctgctg gaagttaccg 5160
   cggcgggtgt ttgtcatagc gatgatttca tcatgagcct gccggaagaa cagtatacct 5220
   atggcctgcc gctgaccctg ggtcatgaag gtgcgggtaa agttgcggcg gttggcgaag 5280
   gtgttgaagg cctggatatc ggcaccaacg tggtggttta tggtccgtgg ggttgcggta 5340
   attgctggca ttgtagccag ggcctggaaa actattgtag ccgtgcgcag gaactgggca 5400
   tcaatccgcc gggtctgggt gcgccgggtg cgctggcgga attcatgatt gtggatagcc 5460
   cgcgtcatct ggttccgatt ggcgatctgg atccggtgaa aaccgttccg ctgaccgatg 5520
   cgggtctgac cccgtatcat gcgatcaaac gcagcctgcc gaaactgcgt ggtggtagct 5580
   atgcggtggt tattggcacc ggtggtctgg gtcatgttgc gattcagctg ctgcgtcatc 5640
   tgagcgcggc gaccgttatt gcgctggatg tgagcgcgga taaactggaa ctggcgacca 5700
   aagttggtgc gcatgaagtg gtgctgagcg ataaagatgc ggcggaaaac gtgcgcaaaa 5760
   tcaccggtag ccagggtgcg gcgctggttc tggattttgt gggctaccag ccgaccattg 5820
   ataccgcgat ggcggttgcg ggtgttggta gcgatgtggc gattgtgggc attggtgatg 5880
   gtcaggcgca tgcgaaagtg ggtttcttcc agagcccgta tgaagcgagc gttaccgttc 5940
   cgtattgggg tgcgcgcaac gaactgattg aactgatcga tctggcgcat gcgggcatct 6000
   ttgatatcgc ggtggaaacc ttcagcctgg ataatggcgc ggaagcgtat cgtcgtctgg 6060
   cggcgggcac cctgagcggt cgtgcggttg ttgttccggg tctgtaagct tctgcatcga 6120
   tcaccacaat tcagcaaatt gtgaacatca tcacgttcat ctttccctgg ttgccaatgg 6180
   cccattttcc tgtcagtaac gagaaggtcg cgaattcagg cgctttttag actggtcgta 6240
   atgaacaatt cttaagaagg agatataca 6269